# EUROPÄISCHE PATENTANMELDUNG

(11) **EP 1 480 152 A2**
(43) Veröffentlichungstag der Anmeldung: **24.11.2004**
(21) Anmeldenummer: 04010586.8
(22) Anmeldetag: 04.05.2004
(51) Int. Cl.: G06F 19/00

(54) **Verfahren und Vorrichtung zur Verarbeitung von Daten eines Therapieinformationen umfassenden Datensatzes**

(30) Priorität: 20.05.2003 DE 10322683
(71) Anmelder: SIEMENS AKTIENGESELLSCHAFT, 80333 München (DE)
(72) Erfinder: Abraham-Fuchs, Klaus, 91058 Erlangen (DE); Rumpel, Eva Dr., 91052 Erlangen (DE); Tiffe, Sven, 1020 Wien (AT)

(57) **Zusammenfassung**

Zur Reduzierung und schnellen Suche von Daten, welche periodisch wiederkehren oder aufeinanderfolgen, z.B. von Daten periodisch wiederkehrender und/oder aufeinander folgender Behandlungen für eine Vermeidung von Mehrfachuntersuchungen an einem Patienten, werden erfindungsgemäß bei einem Verfahren zur Verarbeitung von periodisch wiederkehrenden oder aufeinander folgenden Daten diese auf mindestens ein Periodizitätskriterium (P) geprüft, welches identifiziert und mittels einer Zeitfunktion derart verarbeitet wird, dass anhand des Periodizitätskriteriums (P) und der Zeitfunktion im betreffenden Datensatz (DS) die nächstfolgenden Daten (D), insbesondere die Daten (D) einer nächstfolgenden Behandlung (2n+1), ermittelt und ausgegeben werden.

## Beschreibung

Die Erfindung betrifft ein Verfahren zur Verarbeitung von Daten eines Therapieinformationen umfassenden Datensatzes, welcher periodisch wiederkehrende oder aufeinander folgende Behandlungen bei einem Patienten anhand der Therapieinformationen in Form der Daten umfasst. Des Weiteren betrifft die Erfindung eine Vorrichtung und ein Computerprogrammprodukt zur Durchführung des Verfahrens. Der Datensatz wird im Folgenden kurz selbst als Therapieinformation bezeichnet. Der Datensatz umfasst als Daten Therapieinformationen. Im allgemeinen sind in einer Datenbank mehrere Datensätze für verschiedene Therapieinformationen eines oder mehrerer Patienten in Art einer elektronischen Patientenakte (auch kurz EPR genannt) hinterlegt. Dabei kann die elektronische Patientenakte über Datenbanken mehrerer Institutionen des Gesundheitswesens, z. B. mehrerer behandelnder Ärzte oder Krankenhäuser verteilt sein.

Bei der Behandlung eines Patienten kann es vorkommen, dass gleiche oder mehrere Behandlungen für verschiedene Krankheitsbilder oder von verschiedenen Ärzten in gleichen oder unterschiedlichen periodischen Abständen gefordert bzw. angefordert werden. Hierbei kann es bedingt durch unterschiedliche Krankheitsbilder, welche häufig von verschiedenen Fachärzten behandelt werden, zu mehrfachen gleichartigen Untersuchungen kommen. Dies führt zu hohen Behandlungskosten und ist darüber hinaus auch für den Patienten häufig mit einem enormen Zeitaufwand verbunden.

Zur Lösung dieser Probleme von mehrfachen gleichartigen Verordnungen und daraus resultierenden parallelen gleichartigen Behandlungen sind nach bestem Wissen der Anmelderin im Stand der Technik bisher keine automatisch ablauffähigen Verfahren bekannt geworden. Zudem ist der breite Einsatz von in elektronischer Form hinterlegten Therapieinformationen (= Patientendaten) und der vernetzte Zugriff darauf in der Medizin erst im Entstehen begriffen.

Der Erfindung liegt daher die Aufgabe zugrunde, die Anzahl der Daten für eine Auswahl oder eine Ausgabe möglichst weitgehend zu reduzieren, wobei insbesondere periodisch wiederkehrende Daten reduziert werden sollen, so dass bei einer Anwendung auf Daten für eine Behandlung von Patienten unnötige und mehrfache gleichartige Behandlungen bei einem Patienten reduziert oder gar vermieden können, insbesondere periodisch wiederkehrende oder aufeinander folgenden Behandlungen optimiert werden können.

Diese Aufgabe wird erfindungsgemäß mit den Merkmalen des Anspruchs 1 gelöst. Dazu ist bei einem Verfahren zur Verarbeitung von Daten eines Therapieinformationen umfassenden Datensatzes, welcher periodisch wiederkehrende oder aufeinander folgende Behandlungen bei einem Patienten anhand der Therapieinformationen in Form der Daten umfasst, vorgesehen, dass die in einem einzelnen Datensatz oder die in miteinander verknüpften Datensätzen hinterlegten Daten auf mindestens ein Periodizitätskriterium geprüft werden, welches identifiziert und mittels einer Zeitfunktion derart verarbeitet wird, dass anhand des Periodizitätskriteriums und der Zeitfunktion im betreffenden Datensatz oder in einem verknüpften Datensatz die nächstfolgenden Daten, insbesondere die nächstfolgenden Daten einer nächstfolgenden Behandlung, automatisch ermittelt und ausgegeben werden.

Unter einer Behandlung wird hierbei eine Folge von diagnostischen Untersuchungen und/oder therapeutischen Maßnahmen verstanden, die wiederum in einzelne Behandlungsschritte unterteilt werden können. Ein Behandlungsschritt kann also wiederum eine diagnostische Untersuchung oder eine therapeutische Maßnahme darstellen. Zur Vereinfachung wird im folgenden nur von Behandlungen oder einer Behandlungsfolge gesprochen.

Ein Datensatz von Therapieinformationen umfasst dabei in seiner allgemeinsten Form eine Sammlung von Daten, wie Patientendaten und/oder Daten über bereits ausgeführte und/oder noch auszuführende Therapien oder Untersuchungen und/oder allgemeine Daten wie Daten über diagnostische und/oder therapeutische Mittel. Eine Behandlung kann dabei eine Untersuchung am Patienten und/oder eine am Patienten durchzuführende Therapie sein. Häufig ist dabei ein Bestandteil einer Therapieinformation etwa eine Behandlung, insbesondere eine Untersuchung, ein zugehöriger Zeitpunkt, z. B. ein Datum, an dem die Behandlung, d. h. in diesem Fall die Untersuchung durchgeführt wurde oder werden soll. Anhand von als Daten hinterlegten Ausführungszeitpunkten für Untersuchungen oder Therapien während einer Behandlungsfolge wird nunmehr vorteilhafterweise ein Periodizitätskriterium identifiziert, welches zur Ermittlung der nächstfolgenden Behandlung verwendet wird. Alternativ oder zusätzlich kann anhand anderer Daten mit periodischem Charakter oder Zeitcharakter - wie beispielsweise einer Anzahl von erforderlichen Nachsorgeuntersuchungen oder Paralleluntersuchungen - ein zugehöriges Periodizitätskriterium, wie z. B. Wochen-, Monats- oder halbjährlicher Zyklus für periodisch wiederkehrender Behandlungen bzw. vereinbarte Untersuchungstermine, ermittelt werden. Mit anderen Worten: Ein Periodizitätskriterium kann dabei beispielsweise ein Algorithmus sein, der mittels eines Parameters, wie z. B. "wöchentlich", "monatlich", "jährlich" regelmäßig auftretende Behandlungsschritte einer Behandlungsfolge oder eine Liste von Terminen von sich periodisch wiederholenden oder aufeinander folgenden Behandlungen (auch Behandlungsschritte genannt) beschreibt.

Des Weiteren kann der Datensatz für eine Therapieinformation mit einem weiteren Datensatz für einen Therapiehinweis verknüpft sein. Dabei umfasst der Datensatz für einen Therapiehinweise in seiner allgemeinsten Form eine Sammlung von Daten - wie Eingangs- und/oder Ausgangsvariablen für Therapieinformationen und eine Anzahl von Expertenregeln - anhand derer auf der Basis von Therapieinformationen, z. B. anhand von Patientendaten und/oder anhand von Daten über verfügbare diagnostische und/oder therapeutische Mittel, mittels der Expertenregeln ein Therapiehinweis generiert wird. Die Therapieinformationen werden zur Generierung des Therapiehinweises in Form von Eingangs- oder Nutzdaten bereitgestellt. Darüber hinaus werden anhand von generierten Therapiehinweisen entsprechende Daten, wie beispielsweise der Anzahl von erforderlichen Vorsorge- und/oder Nachsorgeuntersuchungen in den Therapieinformationen hinterlegt. Mit anderen Worten: Therapiehinweise umfassende Datensätze und Therapieinformationen umfassende Datensätze sind miteinander über entsprechende Referenzierungen verknüpft.

Die Erfindung geht von der Erkenntnis aus, dass derzeit eine starke Ausweitung des Einsatzes moderner Informations- und Kommunikationstechnologie im Gesundheitswesen stattfindet. Der Einsatz elektronischer Datenverarbeitung im Krankenhaus (z. B. HIS = Hospital Information System, RIS = Radiology Information System, PACS = Picture Archive & Communication System, LIS = Laboratory Information System) und in der Arztpraxis (Praxisverwaltungssoftware, elektronische Patientenakte) wird mehr und mehr üblich. Von einem folgenden Entwicklungsschritt wird allgemein erwartet, dass eine Vernetzung dieser Software und Datenbanken über die Institutionen des Gesundheitswesens (Kliniken, Arztpraxen, Therapeutenpraxen etc.) hinweg stattfinden wird. Damit wird die Möglichkeit für ein "Vernetztes Gesundheitswesen", zuerst auf nationaler oder regionaler Ebene und später global geschaffen. Diese Entwicklung bietet die Basis zum Einsatz der beiden Aspekte der Erfindung.

Der Vorteil der Erfindung und ihrer Ausgestaltungen besteht insbesondere darin, dass komplette Behandlungsprozesse mit individuellen Behandlungsabfolgen auf periodisch wiederkehrende oder aufeinander folgende Behandlungsschritte prüfbar und überwachbar sind.

Vorteilhafte Ausgestaltungen der Erfindung sind Gegenstand der Unteransprüche.

Zweckmäßigerweise wird anhand von neu eingegebenen Therapieinformationen mindestens ein Periodizitätskriterium identifiziert. Dies ermöglicht eine automatische und sofortige Erkennung von periodisch wiederkehrenden Behandlungen und/oder von zeitlich nahe bei einander liegenden Behandlungen bei einem Patienten, welche ggf. jeweils als Voraussetzungen gleiche Untersuchungsergebnisse benötigen, so dass zum einem periodisch wiederkehrende, aber auch aufeinander folgende, für verschiedene Krankheitsbilder auszuführende Behandlungen dahingehend optimiert werden können, dass die Anzahl von durchzuführenden Untersuchungen und/oder therapeutischen Maßnahmen minimiert wird. Beispielsweise kann für mehrere Behandlungen bei einem Patienten jeweils eine Blutuntersuchung erforderlich sein, so dass bei zeitlich nahe zueinander liegenden Behandlungen daher die Anzahl der Blutabnahmen im günstigsten Fall auf eine einzige Blutabnahme reduziert werden kann.

In einer weiteren bevorzugten Ausführungsform des Verfahrens wird anhand des Periodizitätskriteriums für die nächstfolgende Behandlung ein zugehöriger Ausführungszeitpunkt ermittelt. Dies ermöglicht eine automatische Überwachung und Überprüfung von an einem Patienten durchzuführenden Behandlungen. Beispielsweise können hierdurch automatisch Terminvergaben von zeitlich weit vorausliegenden Behandlungen für beispielsweise Vor- und/oder Nachsorgeuntersuchungen besonders einfach verwaltet und automatisch gehandhabt werden.

Zweckmäßigerweise wird für mehrere periodisch wiederkehrende oder zeitlich aufeinander folgende Behandlungen anhand des jeweils zugehörigen Periodizitätskriteriums ein gemeinsamer nächstfolgender Ausführungszeitpunkt ermittelt. Hierdurch ist sichergestellt, dass bei einem Patienten mehrere Untersuchungen oder therapeutische Maßnahmen in der Art einer Visite bei einer einzigen Behandlung durchgeführt werden.

In einer weiteren vorteilhaften Ausführungsform werden für die nächstfolgende Behandlung mindestens eine vorangegangene Behandlung und deren zugehörige Therapieinformationen ermittelt. Je nach Art und Zeitpunkt der Erfassung der Therapieinformationen der vorangegangenen Behandlung können diese beispielsweise für die nächstfolgende Behandlung verwendet werden, so dass ein nochmaliges Eingeben dieser Therapieinformationen oder ein nochmaliges Erfassen und eine hierzu ggf. erforderliche Untersuchung sicher vermieden sind. Dies ist sowohl kostensparend als auch zeitsparend.

Vorteilhafterweise werden die für die nächstfolgende Behandlung zugehörigen erforderlichen Untersuchungsergebnisse ermittelt, wobei anhand der Therapieinformationen der vorangegangenen Behandlung vorangegangene und mit den erforderlichen Untersuchungsergebnissen korrespondierende Untersuchungsergebnisse identifiziert werden. Bevorzugt werden dabei bei Gleichheit von erforderlichen Untersuchungsergebnissen mit vorangegangenen korrespondierenden Untersuchungsergebnissen diese an Stelle einer erneuten Ermittlung von erforderlichen Untersuchungsergebnissen verwendet. Mit anderen Worten: Vorangegangenen Untersuchungsergebnisse, welche auch für die nächstfolgende Behandlung erforderlich sind, werden wieder verwendet. Ein erneutes Erfassen ist somit sicher vermieden.

Zweckmäßigerweise wird anhand der nächstfolgenden Behandlung eine Meldung ausgegeben. Beispielsweise wird je nach Art und Einsatz des Verfahrens bei einer automatischen Identifizierung der nächstfolgenden Behandlung eine Meldung in Form einer elektronischen Nachricht z. B. an den behandelnden Hausarzt und/oder an weitere behandelnden Fachärzte bzw. Klinikärzte ausgegeben. Alternativ oder zusätzlich wird anhand der nächstfolgenden Behandlung eine Meldung für die vorangegangene Behandlung ausgegeben. Beispielsweise wird dabei eine Meldung an den Arzt, der die vorangegangene Behandlung verordnet hat, in Form einer Mitteilung, z. B. als elektronische Nachricht ausgegeben. Alternativ oder zusätzlich wird anhand der erforderlichen Untersuchungsergebnissen eine Meldung ausgegeben. Hierdurch kann beispielsweise eine Meldung in Form einer elektronischen Nachricht von dem die erforderlichen Untersuchungsergebnisse erhebenden Arzt an den die Behandlung verordneten Arzt ausgegeben werden, für den Fall, dass dieser ein anderer ist.

Bei einer weiteren alternativen oder zusätzlichen Ausführungsform des Verfahrens wird anhand von ermittelten Untersuchungsergebnissen eine Meldung ausgegeben. Bevorzugt wird bei Über- und/oder Unterschreitung eines Schwellwertes für mindestens ein Untersuchungsergebnis eine Meldung ausgegeben. Wird beispielsweise ein kritischer Wert bei einer Blutuntersuchung überschritten, so kann automatisch bei Identifizierung der Über- und/oder Unterschreitung eines Schwellwertes eine Meldung an den behandelnden Arzt ausgegeben werden.

Zweckmäßigerweise werden für die jeweilige Behandlung erforderliche Untersuchungsergebnisse lokal und/oder zentral erfasst. Beispielsweise werden die Untersuchungsergebnisse lokal in einem Labor ermittelt und können von dort an ein Institut oder den behandelnden Arzt weitergeleitet werden. Darüber hinaus werden die für die Behandlung erfassten Untersuchungsergebnisse vorzugsweise lokal und/oder zentral hinterlegt werden. Dabei werden beispielsweise die Untersuchungsergebnisse beim behandelnden Hausarzt oder Facharzt und/oder zentral in einem Archiv einer Klinik hinterlegt werden, so dass mehrere Fachärzte auf die erforderlichen Untersuchungsergebnisse zugreifen können. Zusätzlich werden auch die Behandlungen - vorangegangene und/oder nächstfolgende - für eine möglichst genaue, sichere und dauerhafte Dokumentation zentral und/oder lokal hinterlegt.

Für eine sichere und schnelle Identifizierung von behandelnden Ärzten wird der jeweiligen Behandlung vorzugsweise mindestens ein Nutzer, beispielsweise der behandelnde Arzt und/oder das ausführende Labor zugeordnet. Anhand der Zuordnung des Nutzers zu der betreffenden Behandlung können vorzugsweise die für die Behandlung ermittelten Untersuchungsergebnisse automatisch an den Nutzer weitergeleitet werden. Zusätzlich oder alternativ können der jeweiligen Behandlung auch eine Anzahl von Grunddaten zugeordnet werden. Vorzugsweise werden dabei als Grunddaten mindestens Untersuchungsergebnisse, Ausführungszeitpunkt und/oder Ausführungsperiode hinterlegt.

Bezüglich der Vorrichtung zur Durchführung des oben beschriebenen Verfahrens wird die Aufgabe erfindungsgemäß dadurch gelöst, dass ein Computer vorgesehen ist, auf welchem das Verfahren mittels eines Computerprogrammprodukts implementiert ist. Zum Austausch von Informationen, wie Untersuchungsergebnissen oder zur Weiterleitung von Meldungen sind dabei vorzugsweise mehrere Computer, beispielsweise mehrere dem jeweiligen Arzt und/oder einem Labor zugeordnete Computer miteinander vernetzt oder stehen in einer Kommunikationsverbindung. Alternativ kann die Vorrichtung mehrere in einer Klinik vernetzte Computer mit einem Computerprogrammprodukt zur Durchführung des Verfahrens umfassen.

Nachfolgend wird ein Ausführungsbeispiel der Erfindung anhand der Zeichnung näher erläutert. Darin zeigen:
- FIG 1: eine schematische Darstellung der softwaremäßigen Realisierung eines mindestens eine Therapieinformation umfassenden Datensatzes, und
- FIG 2: eine schematische Darstellung einer Variante der Therapieinformation gemäß FIG 1.

Einander entsprechende Teile sind in allen Figuren mit den gleichen Bezugszeichen versehen.

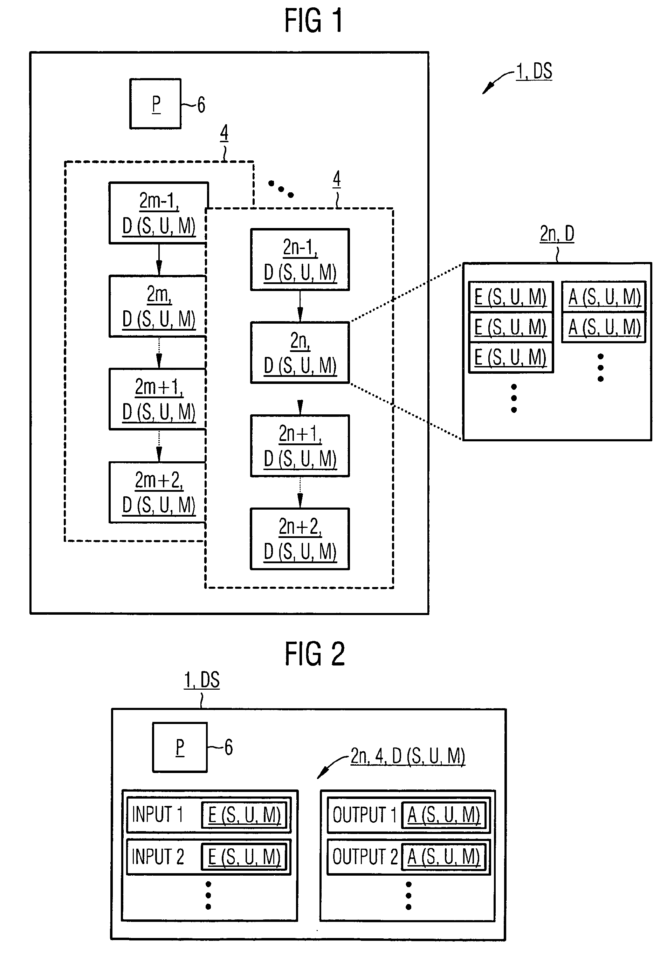

FIG 1 zeigt schematisch einen auf Seiten einer medizinischen Institution, d. h. zum Beispiel in einer Arztpraxis oder in einer Klinik, für jeden Patienten oder ggf. für eine Gruppe von Patienten geführten und Therapieinformationen 1 umfassenden Datensatz DS. Ein solcher Therapieinformationen 1 umfassender Datensatz DS wird im Folgenden kurz auch als Therapieinformation 1 bezeichnet. Eine gebräuchliche Form einer Therapieinformation 1 ist eine elektronische Patientenakte. Die Therapieinformation 1 kann sich im Einzelfall auch auf räumlich verteilte Datenbanken erstrecken. Wichtig ist im Folgenden aber eher das Layout einer solchen Therapieinformation 1 und die jeweils hinterlegten und miteinander verknüpften Informationen und weniger der konkrete Speicherort einzelner oder sämtlicher Informationen oder Daten.

Im Datensatz DS einer jeweiligen Therapieinformation 1 oder elektronischen Patientenakte sind dabei zur Dokumentation von medizinischen Behandlungen 2n, 2n+1, ..., 2n+2 (im Weiteren kurz 2n genannt) entsprechende Daten D hinterlegt. Bedarfsweise sind Daten D über den Beginn, über die bisherige Dauer, über das voraussichtliche oder tatsächliche Ende der jeweiligen medizinischen Behandlung 2n am Patienten gespeichert. Für eine genaue und sichere Dokumentation der medizinischen Behandlung 2n umfasst die Therapieinformation 1 als Daten D zum einen Beschreibungen oder Auflistungen von Symptomen S, Messwerte M oder Untersuchungsergebnisse U und dergleichen und zum anderen auch Therapeutische Maßnahmen und Medikationen und ähnliches.

Mit anderen Worten: Unter einer Behandlung 2n wird im folgenden eine diagnostische Untersuchung und/oder therapeutische Maßnahme verstanden, die jeweils wiederum in einzelne Behandlungsschritte unterteilt werden kann. Ein Behandlungsschritt kann also wiederum eine diagnostische Untersuchung oder eine therapeutische Maßnahme darstellen. Zur Vereinfachung wird im folgenden nur von Behandlungen 2n gesprochen.

Jede einzelne Behandlung 2n ist in einer Behandlungsfolge 4 abgespeichert. Die Behandlungsfolge 4 ist z. B. eine so genannte verkettete Liste von periodisch wiederkehrenden oder zeitlich aufeinander folgenden verschiedenartigen Behandlungen 2n, wobei jedes Listenelement durch eine Behandlung 2n gebildet wird. Mittels der Behandlungsfolge 4 bleiben auch in der Vergangenheit liegende und somit vorangegangene Behandlungen 2n-1 anhand der Daten D dokumentierbar. Zudem ist die historische Entwicklung der jeweiligen Behandlung 2n-1, 2n, 2n+1 jederzeit nachvollziehbar.

Um im Falle von mehreren periodisch wiederkehrenden oder zeitlich aufeinander folgenden Behandlungen 2n diese zu optimieren und Belastungen des Patienten durch Mehrfachuntersuchungen von im Wesentlichen gleichartigen Behandlungen 2n zu vermeiden, wird anhand der Daten D der Therapieinformation 1 mindestens ein Periodizitätskriterium P ermittelt, anhand dessen eine nächstfolgende Behandlung 2n+1 ermittelt wird. Dazu wird der betreffende Datensatz DS auf Daten D mit periodischen Charakter oder Zeitcharakter, zum Beispiel auf einen Ausführungszeitpunkt (= Termin oder Beginn einer der Behandlungen 2n, 2n+1), untersucht. Alternativ oder zusätzlich wird anhand von neuen Eintragungen, z. B. anhand von in der Therapieinformation 1 einzutragenden Eingangsdaten E wie eine Verordnung eines Arztes und/oder Ausgangsdaten A einer vorangegangenen Behandlung 2n-1, automatisch ein Periodizitätskriterium P ermittelt. Anhand des ermittelten Periodizitätskriteriums P wird dann für die nächstfolgende Behandlung 2n+1 ein zugehöriger Ausführungszeitpunkt identifiziert. Unter Periodizitätskriterium P kann hierbei ein Algorithmus verstanden werden, der mittels eines Parameters im Datensatz DS wie z. B. "wöchentlich", "monatlich" ein regelmäßiges Auftreten einer Behandlung 2n oder eines Behandlungsschritts in einer Behandlungsfolge 4 beschreibt.

Beispielsweise wird hierzu unter Verwendung der so genannten Software-Agenten 6 die häufig räumlich auf verschiedene Computer verteilte Therapieinformation 1 (= elektronische Patientenakte) auf die Ausführungszeitpunkte von nächstfolgenden Behandlungen 2n+1, 2n+2 (im Weiteren kurz 2n+1 genannt) untersucht, indem beispielsweise in Art eines Rasters von Untersuchungsperioden oder Untersuchungsabständen, d. h. Ausführungszeitpunkt und Zeitabstand, mehrere zukünftige nächstfolgende Behandlungen 2n+1 miteinander auf gleichartige oder gleiche Untersuchungen verglichen werden. Die Aufgabe von Software-Agenten 6 ist es, das periodische Auftreten von Behandlungen 2n im Datensatz DS zu finden und diese daraufhin zu überprüfen, ob dieselbe Behandlung 2n auch periodisch auftritt. Falls ja, wird der Software-Agent 6 die Termine der einzelnen Behandlungen 2n derart optimieren, dass so wenig medizinische Untersuchungen und/oder therapeutische Maßnahmen als möglich durchgeführt werden müssen. Die nachfolgend beschriebenen Verfahren zur Identifizierung von periodisch wiederkehrenden Behandlungen 2n und nächstfolgenden Behandlungen 2n+1, 2n+2 wird anhand von Software-Agenten 6 ausgeführt. Die Software-Agenten 6 sind dabei Software-Module, welche in der elektronischen Patientenakte in einem separaten Datensatz DS oder in einem auf mehrere Datenbanken in einem Datennetz verteilten Datensatz DS selbständig und somit automatisch anhand des Periodizitätskriteriums P nach Informationen oder Daten D im Datensatz DS suchen, wodurch wie oben beschrieben periodisch wiederkehrende und/oder zeitlich aufeinander folgende Behandlungen 2n hinsichtlich der Vermeidung von Mehrfachuntersuchungen optimiert werden.

Beispielsweise sind aufgrund einer Diagnose bestimmte nächstfolgende Behandlungen 2n+1, insbesondere Untersuchungen und/oder therapeutische Maßnahmen, halbjährlich im Januar und Juli auszuführen, aufgrund einer anderen Diagnose sind weitere nächstfolgende Behandlungen 2m+1, z. B. vierteljährliche Untersuchungen im Februar, Mai, August und November erforderlich. Um zum einen den Patienten zu entlasten und zum anderen auch Kosten zu sparen, können daher in diesem Fall die Untersuchungen bzw. therapeutische Maßnahmen beider Behandlungen 2n+1 und 2m+1 dahingehend optimiert werden, dass diese auf Gleichartigkeit, insbesondere auf möglichst identische Untersuchungen geprüft werden. Hierdurch ist sichergestellt, dass obwohl den verschiedenen Diagnosen unabhängig voneinander nächstfolgende Behandlungen 2n+1 und 2m+1 zugeordnet wurden, diese bei einmal identifiziertem Vorliegen identischer Untersuchungen oder Gleichartigkeit zusätzlich auf einen gemeinsamen Ausführungszeitpunkt hin gesichtet und überprüft werden. Beispielsweise werden bei identifizierten gleichartigen Untersuchungen die vierteljährlichen Untersuchungen um einen Monat verschoben, so dass diese mit den halbjährlichen Untersuchungen übereinstimmen und lediglich noch Untersuchungen im Januar, April, Juli und September ausgeführt werden. Dazu umfasst ein betreffender Software-Agent in Form eines Algorithmus Zeitfunktionen, insbesondere Quotienten, Intervalle, Mindestzeitabstände u. ä. anhand derer die Daten D im Datensatz DS auf Gleichheit oder auf innerhalb eines Toleranzbereiches liegende Werte überprüft werden.

Während einer jeden Behandlung 2n und/oder 2m können dabei neue Daten D z. B. Symptome S und Untersuchungsergebnisse U erhoben werden. Diese Daten D können als Eingangs- und Ausgangsdaten E bzw. A der jeweiligen Behandlung 2 im Datensatz D der Therapieinformation 1 entweder direkt oder indirekt, d. h. mittels einer Referenzierung des eigentlichen Ausgabe- oder Speicherortes, eingegeben bzw. ausgegeben und abgespeichert werden. Dadurch können, wenn z. B. in mehreren aufeinander folgenden Behandlungen 2n und/oder 2m immer wieder gleiche Ausgangsdaten A generiert werden oder immer wieder gleiche oder zumindest teilweise gleiche Eingangsdaten E aufgenommen werden, Mehrfacheintragungen verhindert werden, indem gleiche Eingangs- oder Ausgangsdaten E, A mehrfach einen Speicherplatz mit dem jeweils gleichen Eingangs- oder Ausgangsdatum E bzw. A referenzieren. Mit anderen Worten: Für die nächstfolgende Behandlung 2n+1 und/oder 2m+1 werden mindestens eine der vorangegangenen Behandlungen 2n-1 und/oder 2m-1 und deren zugehörigen Daten D, insbesondere deren Untersuchungsergebnisse U ermittelt, wobei anhand der Daten D bereits vorhandene Untersuchungsergebnisse U einer vorangegangenen Behandlung 2n-1 und/oder 2m-1 als Untersuchungsergebnisse für die nächstfolgende Behandlung 2n+1 wieder verwendet werden können.

Da je nach Grad und Umfang der Dokumentation der jeweiligen medizinischen Behandlung 2n und/oder 2m die Anzahl der zur Verfügung stehenden Eingangsdaten E und/oder die Anzahl der Ausgangsdaten A variieren kann, können zudem die Eingangs- und/oder die Ausgangsdaten E bzw. A beispielsweise in einer Eingangs- bzw. Ausgangsdatenliste (nicht dargestellt), insbesondere in Form einer verketteten Liste, abgelegt sein. Dies gestattet eine dynamische, bedarfsgerechte Bereitstellung von Speicherplatz für genau so viele Eingangs- und Ausgangsdaten E bzw. A, wie bei der konkreten Behandlung 2n zur Verfügung stehen darf bzw. generiert werden.

Die Datenstruktur der Therapieinformation 1 wird in einer Darstellung, wie in FIG 2 dargestellt, auf einer Benutzeroberfläche eines Arbeitsplatzrechners, also z. B. einem von einem Arzt in seinem Sprech- oder Behandlungszimmer verwendeten Personal Computer angezeigt. Die Eingangsdaten E wie Messdaten, z. B. EKG-Daten können entweder manuell oder mittels gebräuchlicher "Drag-and-Drop"-Techniken in die Therapieinformation 1 eingetragen werden. Es ist auch möglich, dass anstelle der eigentlichen Eingangsdaten E eine Referenz auf den Speicherort der Eingangsdaten E eingetragen wird. Bei einer Auswertung der Therapieinformation 1 durch den Arzt oder Therapeuten kann darüber hinaus eine Eintragung von Ausgangsdaten A, z. B. in Form einer Meldung M, aus dem Datensatz DS zur Weiterleitung an andere Nutzer, z. B. an einen Facharzt ausgeführt werden. Diese Eintragung kann je nach Art und Funktion der Ausgangsdaten A automatisch oder manuell ausgeführt werden. Beispielsweise kann als Meldung M automatisch eine elektronische Nachricht an einen weiteren den selben Patienten behandelnden Arzt gesendet werden.

Über den oder jeden Eingang E werden der Therapieinformation 1, Daten D wie z. B. Patientendaten oder Informationen, über medizinische Untersuchungen und/oder therapeutische Maßnahmen und somit über Behandlungen 2 und/oder diagnostische und/oder therapeutische Mittel, direkt oder indirekt zugeführt.

Der behandelnde Mediziner, also der Arzt oder Therapeut, kann dabei die Eingangsdaten E in die jeweilige Therapieinformation 1 manuell eintragen. Alternativ können die Eingangsdaten E auch automatisch von einem die Untersuchung ausführenden medizinischen Gerät übertragen werden. Bei automatischer Anwendung der Therapieinformation 1 können dann anhand der Eingangsdaten E Ausgangsdaten A generiert werden, die dann zum einen auch automatisch in die Therapieinformation 1 eingetragen und zum anderen automatisch in Form einer Meldung M weitergeleitet werden.

Darüber hinaus kann der Mediziner beispielsweise bei der Dokumentation oder zur Weiterleitung einer Information über seine verordneten oder bereits ausgeführten Behandlungen 2n-1 und/oder 2m-1 manuell definierbare Eingangs- und/oder Ausgangsdaten E bzw. A im Datensatz DS, z. B. Zusammenstellen von Verteilerlisten der an einer vorgegebenen Behandlung 2n interessierten Nutzern als Ausgangsdaten A bei vorgegebenen Eingangsdaten E, definieren. Diese frei definierbaren Ein- und Ausgangsdaten E, A werden in der jeweiligen Therapieinformation 1 gespeichert und können an eine nicht näher dargestellte und eine zentrale Datenbank betreibende Institution zur Archivierung oder Dokumentation oder auch zur Abrechnung gegenüber anderen Institutionen weitergeleitet werden.

Darüber hinaus kann zudem bei einer über mehrere Datenbanken verschiedener Institutionen verteilten elektronischen Patientenakte eine Identifizierung von zurückliegenden Behandlungen 2n-1 und/oder 2m-1, wie Untersuchungen, therapeutische Maßnahmen, Diagnosestellungen und Therapieentscheidungen zur Berücksichtigung von dabei erhobenen Untersuchungsergebnissen U oder Symptomen S bei nachfolgenden Behandlungen 2n+1 und/oder 2m+1 oder zur Benachrichtigung von betreffenden Nutzern ausgeführt werden. Bevorzugt wird bei einer neuen Diagnosestellung, nach jeder Überweisung an einen weiterbehandelnden Arzt, bei jedem neuen Eintrag in die Patientenakte, an periodisch wiederkehrenden und somit zu vorgegebenen oder vorgebbaren, insbesondere äquidistanten Ausführungszeiten oder nach jeder Aktualisierung der Patientenakte durch neue Untersuchungsergebnisse U oder therapeutische Maßnahmen und somit zeit- und/oder ereignisgesteuert eine Identifizierung von zurückliegenden Behandlungen 2n-1 und/oder 2m-1 ausgeführt. Hierbei wird vorzugsweise automatisch bei einer neuen Verordnung einer zukünftigen nächstfolgenden Behandlung 2n+1 und/oder 2m+1 oder bei einer aufgrund von periodischen Daten D automatisch identifizierten nächstfolgenden Behandlung 2n+1 und/oder 2m+1 eine Meldung M beispielsweise in Form einer elektronischen Nachricht als Ausgangsdatum A an einen weiteren Nutzer, z. B. an einen Arzt einer vorangegangenen Behandlung 2n-1 bzw. 2m-1, an ein Labor oder ein Institut, welches zur Erhebung von Untersuchungsergebnissen beauftragt wird, ausgegeben.

Des Weiteren können im Datensatz DS Einträge vorgenommen werden, mit welcher automatisch zum vorgegebenen Zeitpunkt die erfassten Untersuchungsergebnisse U an weitere Nutzer ausgegeben werden. So kann beispielsweise das Untersuchungsergebnis U der jährlichen Messung des Augeninnendrucks durch den Augenarzt automatisch dem Diabetologen zugesandt werden, wenn dieser ebenfalls die Messung verordnet hat. Wie oben bereits beschrieben, werden bei derartig zeitlich nahe aufeinander folgenden Untersuchungen mit gleichen auszuführenden Messungen auf einen gemeinsamen Ausführungszeitpunkt optimiert, so dass diese Untersuchung nur einmal erhoben wird. Bei einem weiteren Beispiel kann bei einem Untersuchungsauftrag von einer Blutprobe, welcher üblicherweise von einem externen Labor ausgeführt wird, automatisch ein Vermerk verknüpft und angefügt sein, dass neben dem Auftraggeber, z. B. dem Hausarzt, die Untersuchungsergebnisse U auch einer anderen ebenfalls eine Blutprobenuntersuchung anfordernden Stelle, z. B. einem Klinikarzt oder einem Facharzt, zugesandt wird. In einer besonders einfachen Ausführungsform wird anstelle der Weiterleitung von Untersuchungsergebnissen U der weiteren anfordernden Stelle lediglich eine Meldung M auf den Eintrag der Untersuchungsergebnisse U in der elektronischen Patientenakte - dem Datensatz DS - zugesandt, auf welche diese Stelle zugreifen kann.

Des Weiteren kann zur Vereinfachung der Arbeitsprozesse und der häufig räumlich weit voneinander entfernten Arztpraxen für den jeweiligen Patienten, in dessen elektronischer Patientenakte (= Datensatz DS) als weitere Daten D eine Liste von Nutzern, z. B. eine Liste aller behandelnder Ärzte, hinterlegt sein. Diese Daten D werden dabei verknüpft mit den betreffenden Nutzer interessierenden Informationen wie aktuelle Untersuchungsergebnisse U. Wird nun von einem der Nutzer, beispielsweise dem Hausarzt ein Untersuchungsergebnis U erhoben, so kann automatisch anhand der Liste der behandelnden Ärzte und der diesen zugeordneten Informationen das aktuelle Untersuchungsergebnis U an jene Nutzer weitergeleitet werden, die diese Untersuchungsergebnisse U ebenfalls benötigen. Alternativ können die aktuellen Untersuchungsergebnisse U zentral gespeichert werden und vom jeweiligen Arzt abgerufen werden. Hierbei wird lediglich bei neu erfassten Untersuchungsergebnissen eine Meldung M an den betreffenden Arzt gesandt.

In einer weiteren bevorzugten Ausführungsform wird jede periodisch wiederkehrende Behandlung 2n und/oder 2m in eine Liste mit einer Anzahl von Daten D (= Grunddaten) hinterlegt. Dabei werden als Grunddaten beispielsweise ein Abstand zwischen zwei Behandlungen 2n und 2n+1 und/oder 2m und 2m+1, Beginn und Ende der gesamten Behandlungsfolge 4, erlaubte Abweichungen vom Ausführungszeitpunkt der jeweiligen Behandlung 2n bzw. 2m, z. B. nächster Ausführungszeitpunkt ist der 1. April 2003 mit der Zusatzinformation spätestens innerhalb der nächsten 4 Wochen, im Datensatz DS der Therapieinformation 1 hinterlegt. Zweckmäßigerweise sind die Daten D strukturiert und genormt oder standardisiert im Datensatz DS hinterlegt. Zur Identifizierung einer nächstfolgenden Behandlung 2n+1 und/oder 2m+1 anhand des Periodizitätskriteriums P sind dabei die Daten D standardmäßig in Form von Kurzwörtern wie wöchentlich, monatlich, jährlich, etc. oder mit dem konkreten Ausführungszeitpunkt hinterlegt.

## Patentansprüche

1. Verfahren zur Verarbeitung von Daten (D) eines Therapieinformationen (1) umfassenden Datensatzes (DS), welcher periodisch wiederkehrende und/oder aufeinander folgende Behandlungen (2n) bei einem Patienten anhand der Therapieinformationen (1) in Form der Daten (D) umfasst, wobei die in einem einzelnen Datensatz (DS) oder die in miteinander verknüpften Datensätzen (DS) hinterlegten Daten (D) auf mindestens ein Periodizitätskriterium (P) geprüft werden, welches identifiziert und mittels eines Zeitfunktion derart verarbeitet wird, dass anhand des Periodizitätskriteriums (P) und der Zeitfunktion im betreffenden Datensatz (DS) oder in einem verknüpften Datensatz (DS) die nächstfolgenden Daten (D), insbesondere die nächstfolgenden Daten (D) einer nächstfolgenden Behandlung (2n+1), automatisch ermittelt und ausgegeben werden.

2. Verfahren nach Anspruch 1, wobei anhand von neu eingegebenen Therapieinformationen (1) mindestens ein Periodizitätskriterium (P) identifiziert wird.

3. Verfahren nach Anspruch 1 oder 2, wobei anhand des Periodizitätskriteriums (P) für die nächstfolgende Behandlung (2n+1) ein zugehöriger Ausführungszeitpunkt ermittelt wird.

4. Verfahren nach einem der vorhergehenden Ansprüche, wobei die Daten (D) für periodisch wiederkehrende oder aufeinander folgende Behandlungen (2n und 2m) anhand des jeweils zugehörigen Periodizitätskriteriums (P) und der Zeitfunktion verarbeitet und ein gemeinsamer nächstfolgender Ausführungszeitpunkt ermittelt wird.

5. Verfahren nach einem der vorhergehenden Ansprüche, wobei die Daten (D) für die nächstfolgende Behandlung (2n+1) anhand des Periodizitätskriteriums (P) und der Zeitfunktion verarbeitet und die Daten (D) mindestens einer vorangegangenen Behandlung (2n-1) und deren zugehörige Therapieinformationen (1) ermittelt werden.

6. Verfahren nach einem der vorhergehenden Ansprüche, wobei für die nächstfolgende Behandlung (2n+1) dieser zugehörige erforderliche Untersuchungsergebnisse (U) ermittelt werden, wobei anhand der Therapieinformationen (1) der vorangegangenen Behandlung (2n-1) vorangegangene und mit den erforderlichen Untersuchungsergebnissen (U) korrespondierende Untersuchungsergebnisse (U) identifiziert werden.

7. Verfahren nach Anspruch 6, wobei bei Gleichheit von erforderlichen Untersuchungsergebnissen (U) mit vorangegangenen korrespondierenden Untersuchungsergebnissen (U) diese an Stelle einer erneuten Ermittlung von erforderlichen Untersuchungsergebnissen (U) verwendet werden.

8. Verfahren nach einem der vorhergehenden Ansprüche, wobei anhand der nächstfolgenden Behandlung (2n+1) eine Meldung (M), insbesondere eine Meldung (M) an einen Nutzer einer vorangegangenen Behandlung (2n-1), ausgegeben wird.

9. Verfahren nach einem der vorhergehenden Ansprüche, wobei anhand der für die nächstfolgende Behandlung (2n+1) erforderlichen Untersuchungsergebnissen (U) eine Meldung (M), insbesondere eine Meldung (M) an ein die Untersuchungsergebnisse (U) erhebenden Institut, ausgegeben wird.

10. Verfahren nach einem der vorhergehenden Ansprüche, wobei anhand von ermittelten Untersuchungsergebnissen (U) für eine Behandlung (2n), insbesondere bei Über- und/oder Unterschreitung eines Schwellwertes für mindestens ein Untersuchungsergebnis (U), eine Meldung (M) ausgegeben wird.

11. Verfahren nach einem der vorangegangenen Ansprüche, wobei die für die jeweilige Behandlung (2n) erforderlichen Untersuchungsergebnisse (U) lokal und/oder zentral erfasst werden.

12. Verfahren nach einem der vorangegangenen Ansprüche, wobei die für die Behandlung (2n) erfassten Untersuchungsergebnisse (U) lokal und/oder zentral hinterlegt werden.

13. Verfahren nach einem der vorangegangenen Ansprüche, wobei der jeweiligen Behandlung (2n) mindestens ein Nutzer, insbesondere ein behandelnder Arzt, zugeordnet wird.

14. Verfahren nach einem der vorangegangenen Ansprüche, wobei die für die jeweilige Behandlung (2n) ermittelten Untersuchungsergebnisse (U), insbesondere an einen Nutzer oder an einen zugeordneten Nutzer weitergeleitet werden.

15. Verfahren nach einem der vorangegangenen Ansprüche, wobei periodisch wiederkehrende oder aufeinander folgende Behandlungen (2n) in einem Speicher hinterlegt werden.

16. Verfahren nach Anspruch 15, wobei periodisch wiederkehrenden oder aufeinander folgenden Behandlungen (2n) eine Anzahl von Grunddaten zugeordnet wird.

17. Verfahren nach Anspruch 16, wobei als Grunddaten mindestens Untersuchungsergebnisse (U), Ausführungszeitpunkt und/oder Ausführungsperiode hinterlegt werden.

18. Vorrichtung zur Durchführung des Verfahrens nach einem der der Ansprüche 1 bis 17, wobei mindestens ein aktiver Software-Agent (6) vorgesehen ist, anhand dessen ein in Form von Daten (D) Therapieinformationen (1) umfassender Datensatz (DS) und dessen Daten (D), insbesondere die periodisch wiederkehrenden und/oder aufeinander folgenden Daten (D), auf mindestens ein Periodizitätskriterium (P) untersucht werden, welches identifiziert und mittels einer Zeitfunktion derart verarbeitet wird, dass anhand des Periodizitätskriteriums (P) und der Zeitfunktion im betreffenden Datensatz (DS) oder in einem verknüpften Datensatz (DS) die nächstfolgenden Daten (D), insbesondere die nächstfolgenden Daten (D) einer nächstfolgenden Behandlung (2n+1), automatisch ermittelt und ausgegeben werden.

19. Computerprogrammprodukt zur Durchführung des Verfahrens nach einem der Ansprüche 1 bis 17, umfassend mindestens einen aktiven Software-Agenten (6), anhand dessen ein in Form von Daten (D) Therapieinformationen (1) umfassender Datensatz (DS) und dessen Daten (D), insbesondere die periodisch wiederkehrenden und/oder aufeinander folgenden Daten (D), auf mindestens ein Periodizitätskriterium (P) untersucht werden, welches identifiziert und mittels einer Zeitfunktion derart verarbeitet wird, dass anhand des Periodizitätskriteriums (P) und der Zeitfunktion im betreffenden Datensatz (DS) oder in einem verknüpften Datensatz (DS) die nächstfolgenden Daten (D), insbesondere die nächstfolgenden Daten (D) einer nächstfolgenden Behandlung (2n+1), automatisch ermittelt und ausgegeben werden.
